# EUROPEAN PATENT APPLICATION

(11) **EP 4 129 232 A1**
(43) Date of publication of application: **08.02.2023**
(21) Application number: 21775834.1
(22) Date of filing: 20.01.2021
(51) Int. Cl.: A61C 13/08, A61C 13/09, A61C 13/00, A61C 13/087, A61C 13/083, B33Y 70/10, B33Y 40/20

(54) **DENTAL PROSTHESIS HAVING STRUCTURE SIMILAR TO THAT OF NATURAL TEETH, AND MANUFACTURING METHOD THEREFOR**

(30) Priority: 26.03.2020 KR 20200036682
(71) Applicant: Hass Co., Ltd., Gangwon-do 25452 (KR)
(72) Inventor: KIM, Yong Su, Gangneung-si Gangwon-do 25497 (KR); OH, Kyung Sik, Incheon 22003 (KR); LIM, Hyung Bong, Ansan-si Gyeonggi-do 15521 (KR); KIM, Joon Hyung, Anseong-si Gyeonggi-do 17560 (KR); KIM, Sung Min, Yongin-si Gyeonggi-do 16903 (KR); SON, Si Won, Seoul 08846 (KR); KIM, Yena, Seoul 08250 (KR)
(74) Representative: Gulde & Partner
(86) International application number: PCT/KR2021/095002
(87) International publication number: WO 2021/194327

(57) **Abstract**

The present disclosure relates to a dental prosthesis having a structure similar to that of natural teeth and a method for manufacturing the same. Proposed is a dental prosthesis capable of expressing a structure and properties similar to those of natural teeth in which enamel is a surface layer and dentine is an inner layer underneath the enamel. In addition, a method of manufacturing the same dental prothesis through three-dimensional printing is also proposed. The dental prosthesis is a cured product including ceramic particles dispersed in a polymer matrix. The cured product includes a first cured product layer including 70% to 90% by weight of ceramic particles having an average particle diameter of 100 to 1,000 nm and a second cured product layer including 40% to 60% by weight of ceramic particles having an average particle diameter of 10 to 500 µm, the second cured product layer being adjacent to the inner surface of the first cured product layer.

## Description

### Technical Field

The present disclosure relates to a dental prosthesis composed of a cured material including ceramic particles dispersed in a polymer matrix and a method of manufacturing the same. More particularly, the present disclosure relates to a dental prosthesis having a structure similar to that of natural teeth and a method of manufacturing the same using a three-dimensional printing technique.

### Background Art

It can be seen that the dental industry is growing together with the development of equipment and materials used in dentistry. For example, the dental industry has advanced due to equipment such as heat-pressing or computer-aided design/computer-aided manufacturing (CAD/CAM), and materials such as glass-ceramic and zirconia, which are suitable materials for these types of equipment, are being developed.

Due to these changes, dentistry has improved the aesthetic demand of patients and has reduced the time for dental treatment due to one-day prosthetics. However, due to the difference in physical properties between natural teeth and dental prosthesis, problems such as the wear of adjacent teeth or antagonist teeth during the chewing process of food may occur, resulting in additional problems such as secondary infection in the oral cavity.

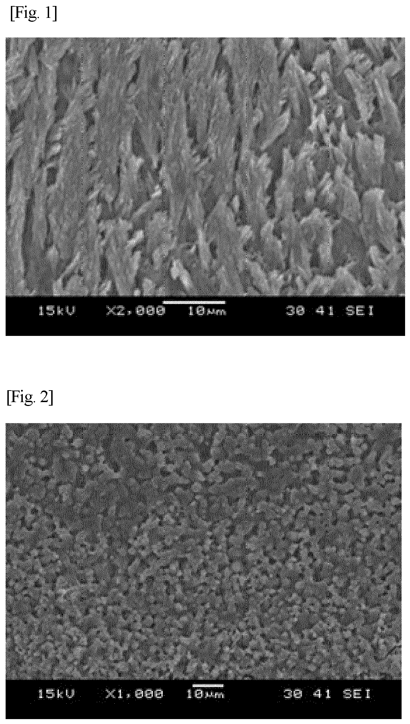

To solve this problem, a lot of research is being conducted on the structure and properties similar to that of natural teeth. In natural teeth, the crown part is composed of enamel and dentin, and each has a different physical property and structure. In the case of enamel, the content of inorganic substances accounts for 85 vol% or more and has a dense structure (see FIG. 1), and in the case of dentin, the ratio of inorganic materials and organic materials is almost similar and has a pore structure (see FIG. 2). Due to these differences in materials and structures, the physical properties of enamel and dentin are also different. For example, in the case of elastic modulus, enamel shows a value of about 50 to 110 GPa, and dentin shows a value of about 20 GPa or less. Also, in the case of hardness, it is reported that enamel exhibits a value of about 3 to 6 GPa, and dentin exhibits a value of about 0.5 to 1.5 GPa.

The development of materials with physical properties and structures most similar to those of natural teeth has been conducted in the past, and various materials have been developed.

As an example, Korea Patent No. 10-2037401 discloses an artificial tooth material exhibiting light transmittance at the level of the enamel of natural teeth. Proposed is a silicate glass based on SiO₂ having a visible light transmittance of 40% to 70% by the heat treatment, in which a silicate glass composition is heat-treated for 1 minute to 2 hours in the 300°C to 600°C section. The silicate glass composition includes 3% to 5% by weight of ZrO₂ to improve wettability for bonding with zirconia, 69% to 79% by weight of SiO₂ serving as a structure of glass, 10% to 13% by weight of Li₂O, 3% to 7% by weight of P₂O₅, 1% to 4% by weight of Al₂O₃, 1.0% to 2.5% by weight of K₂O, 0.1% to 3% by weight of MO (M = any one of Ca, Zn, and Mg), and 0.5% to 2.0% by weight of a colorant. When such SiO₂-based silicate glass is hot-pressed on the outside of the zirconia, the mechanical structural stability of the zirconia prosthesis can be maintained by thin hot-pressing the zirconia prosthesis without cutting the zirconia prosthesis. Therefore, it is described that the color of zirconia comes out through the thin coating layer, thereby obtaining a more dynamic and deep color, thereby producing more similar optical properties to natural teeth.

On the other hand, Korea Patent No. 10-1840142 discloses a method of manufacturing artificial teeth using a dental photocurable resin composition, which includes a radical polymerizable organic compound, a filler, and a photosensitive radical polymerization initiator. Provided is a method to smoothly and easily manufacture artificial teeth in a short time, especially in a short time of less than an hour, by using a dental photocurable resin composition, in which the artificial teeth have all the characteristics required for artificial teeth, such as strength, abrasion resistance, hardness, and low water absorption, and also provides excellent esthetics and functionality.

However, the technology for a prosthesis having a dual structure structurally of enamel and dentin is still insufficient.

### Disclosure

### Technical Problem

An objective of the present disclosure is to provide a dental prosthesis capable of expressing a structure and physical properties similar to those of natural teeth showing a dual structure of enamel and dentin.

In addition, another objective of the present disclosure is to provide a method of manufacturing a dental prosthesis that can express structures and properties similar to those of natural teeth by realizing a dense structure of enamel and a structure with pores of dentin similar to natural teeth using three-dimensional printing, which has a double structure and realizes individual properties.

### Technical Solution

The present disclosure provides a dental prosthesis with a structure similar to that of natural teeth, the dental prosthesis is a cured product including ceramic particles dispersed in a polymer matrix. The dental prosthesis includes: a first cured product layer including 70% to 90% by weight of ceramic particles having an average particle diameter of 100 to 1,000 nm; and a second cured product layer including 40% to 60% by weight of ceramic particles having an average particle diameter of 10 to 500 µm, the second cured product layer being adjacent to the inner surface of the first cured product layer.

In a preferred embodiment of the present disclosure, the first cured product layer may have a biaxial flexural strength of 300 to 500 MPa, an elastic modulus of 50 to 110 GPa, and a hardness of 3 to 6 GPa.

In a preferred embodiment of the present disclosure, the second cured product layer may have a biaxial flexural strength of 100 to 300 MPa, an elastic modulus of 5 to 20 GPa, and a hardness of 0.5 to 1.5 GPa.

In one embodiment of the present disclosure, the ceramic particles may be particles of at least one material selected from the group consisting of barium silicate-based crystallized glass, leucite-based crystallized glass, alumina, zirconia, and glass.

In one embodiment of the present disclosure, the ceramic particles may have a silane-treated surface.

In one embodiment of the present disclosure, the polymer matrix may be a cured product of at least one polymerizable organic compound selected from the group consisting of hydroxy ethyl methacrylate (HEMA), 2,2-bis[4-(2-hydroxy-3-methacryloyloxy propoxy)phenyl]propane (Bis-GMA), triethylene glycol dimethacrylate (TEGDMA), diurethane dimethacrylate (UDMA), urethane dimethacrylate (UDM), biphenyldimethacrylate (BPDM), n-tolylglycine-glycidylmethacrylate (NTGE), polyethylene glycol dimethacrylate (PEG-DMA), and oligocarbonate dimethacrylic esters.

In a preferred embodiment of the present disclosure, the first cured product layer may have a dense structure, and the second cured product layer may have a pore structure.

In one embodiment of the present disclosure, the cured product may be photo-cured or thermal cured.

In a preferred embodiment of the present disclosure, the prosthesis may be manufactured through three-dimensional printing.

In another embodiment of the present disclosure, a method of manufacturing a dental prosthesis having a structure similar to that of natural teeth is provided. In the method of manufacturing a dental prosthesis through three-dimensional printing using a curable composition containing ceramic particles and a polymerizable organic compound, the method includes laminating and curing the composition according to a predetermined shape by using a curable composition, which the curable composition includes: a first curable composition including 70% to 90% by weight of ceramic particles having an average particle diameter of 100 to 1,000 nm; and a second cured product layer including 40% to 60% by weight of ceramic particles having an average particle diameter of 10 to 500 µm.

In the manufacturing method according to an embodiment of the present disclosure, ceramic particles may use particles of at least one material selected from the group consisting of barium silicate-based crystallized glass, leucite-based crystallized glass, alumina, zirconia, and glass.

In one embodiment of the present disclosure, the polymerizable organic compound may use at least one selected from the group consisting of hydroxy ethyl methacrylate (HEMA), 2,2-bis[4-(2-hydroxy-3-methacryloyloxy propoxy)phenyl]propane (Bis-GMA), triethylene glycol dimethacrylate (TEGDMA), diurethane dimethacrylate (UDMA), urethane dimethacrylate (UDM), biphenyldimethacrylate (BPDM), n-tolylglycine-glycidylmethacrylate (NTGE), polyethylene glycol dimethacrylate (PEG-DMA), and oligocarbonate dimethacrylic esters.

In the manufacturing method, according to a preferred embodiment of the present disclosure, the predetermined shape formed from the first curable composition is a dense structure analogous to an enamel of a natural tooth, and the predetermined shape formed from the second curable composition is a pore structure analogous to a dentin of a natural tooth.

In the manufacturing method, according to an embodiment of the present disclosure, curing may be performed by photo-curing or thermal curing.

### Advantageous Effects

The present disclosure proposes a dental prosthesis including ceramic particles dispersed in a polymer matrix and having a structure and properties similar to the enamel and dentin of natural teeth and a method of manufacturing the dental prosthesis using a three-dimensional printing technique. Therefore, it is possible to manufacture dental prostheses with high esthetics and properties similar to adjacent teeth or antagonist teeth required for dental prosthetic materials and can be expected to have the effect of preventing problems such as abrasion of natural teeth with adjacent teeth or antagonist teeth by being placed in the patient's mouth, and secondary infection that may occur as a result.

### Description of Drawings

FIGS. 1 and 2 are SEM photographs showing the microstructure of enamel (FIG. 1) and dentin (FIG. 2) of natural teeth.

### Best Mode

The above and further aspects of the present disclosure will become more apparent through preferred embodiments described with reference to the accompanying drawings. Hereinafter, it will be described in detail so that those skilled in the art can easily understand and reproduce these embodiments of the present disclosure.

FIGS. 1 and 2 are SEM photographs showing the microstructure of enamel (FIG. 1) and dentin (FIG. 2) of natural teeth.

One of the objectives of the present disclosure is maximally realizing such a natural tooth with a double structure through a cured product containing ceramic particles dispersed in a polymer matrix, the present disclosure proposed dental prosthesis including: a first cured product layer including 70% to 90% by weight of ceramic particles having an average particle diameter of 100 to 1,000 nm; and a second cured product layer including 40% to 60% by weight of ceramic particles having an average particle diameter of 10 to 500 µm, the second cured product layer being adj acent to the inner surface of the first cured product layer.

In the present disclosure, it was found that the microstructure difference and physical properties difference between enamel and dentin in natural teeth were influenced by the content and structure of inorganic and organic materials. A curable composition including a polymerizable organic compound and ceramic particles was considered as a material that can structurally express these structures of enamel and dentin, and it was confirmed that the use of three-dimensional printing using the curable composition is optimal for realizing the microstructure of these enamel and dentin. In particular, when two types of curable compositions with controlled ceramic particle sizes and contents are used to implement a double structure, it is confirmed that a cured product close to the enamel and dentin of natural teeth can be obtained by physical properties.

In this aspect, the preferred dental prosthesis includes a first cured product layer including 70% to 90% by weight of ceramic particles having an average particle diameter of 100 to 1,000 nm and a second cured product layer including 40% to 60% by weight of ceramic particles having an average particle diameter of 10 to 500 µm. In this case, the second cured product layer is formed adjacent to the inner side of the first cured product layer, which can be realized by reflecting the layer structure of enamel and dentin of natural teeth, and the shape of the layer is not limited.

In the dental prosthesis of the present disclosure, as the content of the ceramic particles increases and the size of the ceramic particles decreases, the density of the ceramic particles increases after curing, thereby indicating an increase in physical properties.

Specifically, as the size of a ceramic particle is small, and the content of the ceramic particles is high, the properties of the mixture are similar to those of the ceramic, and on the other hand, if the content of the polymer is high, the properties of the mixture are decreased. In addition, even when the content of ceramic is similar to the polymer, the smaller the size of ceramic particles, the higher the distribution of ceramic, which may indicate a change in physical properties.

In this aspect, it is preferable that the content of the ceramic particles included in the first cured product layer is 70% to 90% by weight of the total composition of the first cured product layer, and the ceramic particles included in the first cured product layer have an average particle diameter in a range of 100 to 1,000 nm.

Meanwhile, it is preferable that the content of the ceramic particles included in the second cured product layer is 40% to 60% by weight of the total composition of the second cured product layer, and the ceramic particles included in the second cured product layer have an average particle diameter in a range of 10 to 500 µm.

In the case of including two cured product layers in which the content and size of ceramic particles dispersed in the polymer matrix are controlled as described above, the first cured product layer may exhibit physical properties satisfying a biaxial flexural strength of 300 to 500 MPa, an elastic modulus of 50 to 110 GPa, and the hardness of 3 to 6 GPa, the second cured product layer may exhibit physical properties satisfying a flexural strength of 100 to 300 MPa, an elastic modulus of 5 to 20 GPa, and a hardness of 0.5 to 1.5 GPa. These are equivalent physical properties to the enamel and dentin of natural teeth, respectively. It is possible to realize the physical properties of enamel that is structurally dense and exhibits high physical properties in order to perform the role of chewing food and abutting adjacent teeth and antagonist teeth and the physical properties of dentin with appropriate elasticity and flexibility to relieve the stress that occurs in the chewing process for food.

In the above and below, the "ceramic particles" may include various inorganic materials that can be used in the manufacture of dental prosthesis but are not limited thereto as a specific example, is at least one selected from the group consisting of barium silicate-based crystallized glass, leucite-based crystallized glass, alumina, zirconia, and glass.

On the other hand, the ceramic particles may be desirable in that silane treatment on the surface thereof improves the binding force with the polymer matrix and ultimately improves the mechanical properties of the dental prosthesis. Examples of the silane coupling agent that may be used at this time include a silane coupling agent having a reactive functional group such as a (meth)acrylic group, an epoxy group, a vinyl group, an amino group, and a mercapto group, and one or two or more of these may be used, but are not limited thereto.

The polymer constituting a matrix in the dental prosthesis of the present disclosure may be a cured product of a thermally synthetic or photopolymerizable organic compound well known in the art. Although not limited thereto, for example, the polymer may be a cured product of at least one polymerizable organic compound selected from the group consisting of hydroxy ethyl methacrylate (HEMA), 2,2-bis[4-(2-hydroxy-3-methacryloyloxy propoxy)phenyl]propane (Bis-GMA), triethylene glycol dimethacrylate (TEGDMA), diurethane dimethacrylate (UDMA), urethane dimethacrylate (UDM), biphenyldimethacrylate (BPDM), n-tolylglycine-glycidylmethacrylate (NTGE), polyethylene glycol dimethacrylate (PEG-DMA), and oligocarbonate dimethacrylic esters.

According to the present disclosure, in manufacturing a dental prosthesis with a cured product containing ceramic particles dispersed in such a polymer matrix, a prosthesis having a dual structure composed of enamel and dentin similar to that of natural teeth can be manufactured by using three-dimensional printing.

Currently, three-dimensional printing using metal and polymer is widely used for the manufacture of dental prostheses. Three-dimensional printing using metal is a method of manufacturing by stacking melted solution by melting a metal using a laser, and three-dimensional printing using a polymer is a method of stacking a polymer using a photo-curing agent and curing the polymer using a light source.

In three-dimensional printing printing using only ceramics is still under research, and most of them use a method of mixing and curing organic compounds. At this time, important variables include the content ratio of the ceramic to the polymer and the particle size of the ceramic, and through this, the physical properties after curing can also be controlled. In the present disclosure, the content ratio and the particle size of the ceramic particles are controlled as described above, and three-dimensional printing is used, thereby reproducing the natural teeth structure.

In the case of natural teeth, enamel has a dense structure as shown in FIG. 1, and dentin has a pore structure, and more particularly, a tubular pore structure having a size of about 100 to 1,000 nm as shown in FIG. 2. In the present disclosure, in order to similarly implement these natural teeth, the microstructure may be implemented by using three-dimensional printing.

According to the present disclosure, a double structure such as a natural tooth is realized using three-dimensional printing, and changes in physical properties appearing after curing can be given according to the material constituting each structure, the content of each material, and the particle size of the material. Since the dental prosthesis of the present disclosure exhibits colors and physical properties very similar to those of teeth, the dental prosthesis is suitable for use as a dental prosthetic material.

One of the ultimate goals of the dental prosthesis is to show the most similar esthetic and physical properties to natural teeth and to show the most natural harmony with surrounding teeth when placed in the oral cavity.

To this end, the present disclosure proposes a method for manufacturing a dental prosthesis capable of implementing the enamel and dentin of natural teeth by showing a double structure similar to that of natural teeth using a three-dimensional printing method and controlling physical properties by controlling the contents of ceramic and polymer and ceramic particle sizes.

Specifically, a method for manufacturing a dental prosthesis having a structure similar to that of natural teeth, according to the present disclosure, is to manufacture a dental prosthesis through three-dimensional printing using a curable composition including ceramic particles and a polymerizable organic compound. The method includes laminating and curing a curable composition according to a predetermined shape using a curable composition, which the curable composition includes: a first curable composition including 70% to 90% by weight of ceramic particles having an average particle diameter of 100 to 1,000 nm; and a second cured product layer including 40% to 60% by weight of ceramic particles having an average particle diameter of 10 to 500 µm.

Methods such as stereo lithography apparatus (SLA) and digital lighting process (DLP) are suitable for three-dimensional printing methods to manufacture dental prostheses using ceramics having a structure similar to that of natural teeth. This is a method of printing each layer with a curable composition having a structure of enamel and dentin of natural teeth and curing polymerizable organic compounds using light or heat sources to polymerize the compounds. In this case, an example of the light source may have a wavelength of 300 to 600 nm but is not limited thereto.

In order to have similar physical properties of each of the enamel and dentin of natural teeth, various physical properties can be ensured by controlling changes in ceramic particle size and the content of ceramics and polymers. In this aspect, each layer is printed and laminated using a curable composition, the composition including: a first curable composition in which the content of the ceramic particles is 70% to 90% by weight, and the ceramic particles have an average particle diameter of 100 to 1,000 nm; a second curable composition in which the content of the ceramic particles is 40% to 60% by weight, and the ceramic particles have an average particle diameter of 10 to 500 µm.

In this case, as described above, the ceramic particles may use at least one selected from the group consisting of barium silicate-based crystallized glass, leucite-based crystallized glass, alumina, zirconia, and glass, and these ceramic particles have a silane-treated surface.

In addition, the polymerizable organic compound may use at least one selected from the group consisting of hydroxy ethyl methacrylate (HEMA), 2,2-bis[4-(2-hydroxy-3-methacryloyloxy propoxy)phenyl]propane (Bis-GMA), triethylene glycol dimethacrylate (TEGDMA), diurethane dimethacrylate (UDMA), urethane dimethacrylate (UDM), biphenyldimethacrylate (BPDM), n-tolylglycine-glycidylmethacrylate (NTGE), polyethylene glycol dimethacrylate (PEG-DMA), and oligocarbonate dimethacrylic esters.

For example, the ceramic particles treated with silane are mixed with a solution of such a polymerizable organic compound according to the size and content ratio of the ceramic particles of the above-described two types of curable compositions, and each of the curable compositions is put into three-dimensional printing equipment.

Lamination is performed in a predetermined shape using each of these curable compositions, where the predetermined shape is obtained by simulating the enamel of natural teeth and dentin of natural teeth. In the case of the first curable composition, the composition constituting the outside of the tooth has a dense structure similar to the enamel of natural teeth, and in the case of the second curable composition, the composition constituting the interior, similar to the dentin of natural teeth, has a pore structure, specifically, a structure with tubular pores. The two curable compositions are laminated in their respective structures and cured while lamination is proceeding. In the case of the first curable composition, the composition constituting the outside of the teeth has a dense structure by simulating the enamel of natural teeth, and in the case of the second curable composition, the composition constituting the interior of the teeth has a pore structure, specifically tubular pore structure by simulating the dentin of natural teeth. The two curable compositions are laminated in their respective structures and cured while lamination is proceeding.

At this time, the curing may be photo-curing or thermal curing, and in the case of photo-curing, the light source is irradiated to cause photo-curing to occur.

In the case of using a photocurable agent, curing may occur by supplying a required light source, and in the case of a thermosetting agent, curing may be performed by supplying a required temperature.

According to the present disclosure, a prosthesis including a first cured product layer having a microstructure simulating the enamel shown in FIG. 1 and a second cured product layer having a microstructure simulating the dentin shown in FIG. 2 may be obtained using a first curable composition and a second curable composition. The prosthesis may obtain a cured product layer capable of expressing physical properties, as shown in Table 1 below, which is a physical property corresponding to the enamel and dentin of natural teeth, and according to the present disclosure, a dental prosthesis having a structure similar to that of natural teeth may be provided.

**[Table 1]**

| | Biaxial flexural strength (MPa) | Modulus of elasticity (GPa) | Vickers hardness (GPa) |
|---|---|---|---|
| First cured product layer | 300 to 500 | 50 to 110 | 3.0 to 6.0 |
| Second cured product layer | 100 to 300 | 5 to 20 | 0.5 to 1.5 |

Although the present disclosure has been described with reference to the embodiment illustrated in the drawings, this is merely exemplary, and it will be understood that various modifications and equal other embodiments are possible by those skilled in the art.

### Industrial Applicability

The present disclosure relates to a dental prosthesis composed of a cured material including ceramic particles dispersed in a polymer matrix and a method for manufacturing the same. More particularly, the present disclosure relates to a dental prosthesis having a structure similar to that of natural teeth and a method of manufacturing the same using a three-dimensional printing technique.

The present disclosure proposes a dental prosthesis including ceramic particles dispersed in a polymer matrix and having a structure and properties similar to the enamel and the dentin of natural teeth and a method of manufacturing the dental prosthesis using a three-dimensional printing technique. Therefore, it is possible to manufacture dental prostheses with high esthetics and properties similar to adjacent teeth or antagonist teeth required for dental prosthetic materials and can be expected to have the effect of preventing problems such as abrasion of natural teeth with adjacent teeth or antagonist teeth by being placed in the patient's mouth, and secondary infection that may occur as a result.

## Claims

1. A dental prosthesis with a structure similar to that of natural tooth, the dental prosthesis being a cured product comprising ceramic particles dispersed in a polymer matrix,
the dental prosthesis comprises:
a first cured product layer comprising 70% to 90% by weight of ceramic particles having an average particle diameter of 100 to 1,000 nm;
a second cured product layer positioned adj acent to an inner surface of the first cured product layer and comprising 40% to 60% by weight of ceramic particles having an average particle diameter of 10 to 500 µm.

2. The dental prosthesis of claim 1, wherein the first cured product layer has a biaxial flexural strength of 300 to 500 MPa, an elastic modulus of 50 to 110 GPa, and a hardness of 3 to 6 GPa.

3. The dental prosthesis of claim 1, wherein the second cured product layer has a biaxial flexural strength of 100 to 300 MPa, an elastic modulus of 5 to 20 GPa, and a hardness of 0.5 to 1.5 GPa.

4. The dental prosthesis of claim 1, wherein the ceramic particles are particles of at least one material selected from the group consisting of barium silicate-based crystallized glass, leucite-based crystallized glass, alumina, zirconia, and glass.

5. The dental prosthesis of claim 1 or 4, wherein the ceramic particles have a silane-treated surface.

6. The dental prosthesis of claim 1, wherein the polymer matrix is a cured product of at least one polymerizable organic compound selected from the group consisting of hydroxy ethyl methacrylate (HEMA), 2,2-bis[4-(2-hydroxy-3-methacryloyloxy propoxy)phenyl]propane (Bis-GMA), triethylene glycol dimethacrylate (TEGDMA), diurethane dimethacrylate (UDMA), urethane dimethacrylate (UDM), biphenyldimethacrylate (BPDM), n-tolylglycine-glycidylmethacrylate (NTGE), polyethylene glycol dimethacrylate (PEG-DMA), and oligocarbonate dimethacrylic esters.

7. The dental prosthesis of claim 1, wherein the first cured product layer has a dense structure, and the second cured product layer has a pore structure.

8. The dental prosthesis of claim 1, wherein the cured product is photo-cured or thermal cured.

9. The dental prosthesis of claim 1, wherein the dental prosthesis is manufactured through three-dimensional printing.

10. A method of manufacturing a dental prosthesis having a structure similar to that of natural teeth through three-dimensional printing using a curable composition comprising ceramic particles and a polymerizable organic compound, the method comprising:
forming a laminate using having a predetermined shape using the curable composition wherein the curable composition comprises a first curable composition comprising 70 to 90% by weight of ceramic particles having an average particle diameter of 100 to 1,000 nm and a second curable composition comprising 40% to 60% by weight of ceramic particles having an average particle diameter of 10 to 500 µm; and
curing the laminate.

11. The method of claim 10, wherein the ceramic particles are particles of at least one material selected from the group consisting of barium silicate-based crystallized glass, leucite-based crystallized glass, alumina, zirconia, and glass.

12. The method of claim 10, wherein the polymerizable organic compound uses at least one selected from the group consisting of hydroxy ethyl methacrylate (HEMA), 2,2-bis[4-(2-hydroxy-3-methacryloyloxy propoxy)phenyl]propane (Bis-GMA), triethylene glycol dimethacrylate (TEGDMA), diurethane dimethacrylate (UDMA), urethane dimethacrylate (UDM), biphenyldimethacrylate (BPDM), n-tolylglycine-glycidylmethacrylate (NTGE), polyethylene glycol dimethacrylate (PEG-DMA), and oligocarbonate dimethacrylic esters.

13. The method of claim 10, wherein the predetermined shape formed from the first curable composition is a dense structure analogous to an enamel of a natural tooth, and the predetermined shape formed from the second curable composition is a pore structure analogous to a dentin of a natural tooth.

14. The method of claim 10, wherein the curing is performed by photo-curing or thermal curing.
